# EUROPEAN PATENT APPLICATION

(11) **EP 2 886 643 A1**
(43) Date of publication of application: **24.06.2015**
(21) Application number: 13829939.1
(22) Date of filing: 13.08.2013
(51) Int. Cl.: C12N 1/20, C12N 15/09, C12P 7/62

(54) **PRODUCTION OF BIOPLASTICS**

(30) Priority: 14.08.2012 ES 201231305
(71) Applicant: Neol Biosolutions S.A., 18100 Armilla (Granada) (ES)
(72) Inventor: RONCHEL BARRENO, Mª del Carmen, E- 18100 Armilla (Granada) (ES); MARTÍNEZ GARCÍA, Lorena, E- 18100 Armilla (Granada) (ES); GIBERT AMAT, Jordi, E- 18100 Armilla (Granada) (ES); LARA CAMBIL, Armando, E- 18100 Armilla (Granada) (ES); SUÁREZ GONZÁLEZ, Beatriz, E- 18100 Armilla (Granada) (ES); ADRIO FONDEVILA, José Luis, E- 18100 Armilla (Granada) (ES); VELASCO ALVAREZ, Javier, E- 18100 Armilla (Granada) (ES)
(74) Representative: ABG Patentes, S.L.
(86) International application number: PCT/ES2013/070595
(87) International publication number: WO 2014/027129

(57) **Abstract**

The present invention relates to the production of bioplastics in which a microorganism of the *Pseudomonas putida* species has been isolated and identified and has been found to be a super-producer naturally capable of: a) metabolizing different carbon sources, including aromatic derivatives, b) growing at high cellular concentrations, and c) producing a large amount of polyhydroxyalkanoates or 3-hydroxy acids.

## Description

### Field of the Invention

The present invention relates to the production of plastic polymers and the monomers thereof from cultures of a microorganism in the presence of different carbon sources.

### Background of the Invention

Plastics obtained from renewable sources have undergone extensive development in recent years. The European Commission has identified plastics obtained from renewable sources as one of the most important markets in its "Lead Markets Initiative" (Final Evaluation of the Lead Market Initiative, 2011). The high price of petroleum and pollution problems generated by conventional plastics has resulted in the development of new technologies for producing materials with characteristics similar to petrochemical products that offer other recovery and recycling pathways to aid in reducing the environmental impact.

These materials include, among others, polyhydroxyalkanoates (PHAs), polylactones, certain aliphatic polyesters, some polysaccharides and copolymers derived from petroleum (Shen et al. 2010. Biofuels Bioprod. Bioref., 4, 25-40; Gavrilescu et al. 2005. Biotechnol. Adv., 23, 471-499).

PHAs are a family of polyesters having 3, 4, 5 and 6 hydroxy acids synthesized by different groups of microorganisms which accumulate in cells in the form of granules the number and size of which varies between different species, and they serve as compounds for carbon and energy storage (Chen GQ. 2010. Plastics from Bacteria: Natural Functions and applications, GQ Chen Ed., Springer, 17-37). 30% of soil bacteria has the capacity to synthesize PHA (Wu et al., 2000. Acta Polym. Sin. 6, 751-756). Depending on the number of carbon atoms of the monomeric unit, PHAs are classified as short chain PHAs (containing 3-5 carbon atoms) and medium chain PHAs (containing 6-14 carbon atoms).

Polyhydroxyalkanoate biosynthesis depends primarily on the microorganism and on the carbon source used; different metabolic pathways are linked to the formation of hydroxyacyl-CoA molecules, the main PHA precursor. On the other hand, their physicochemical properties vary significantly depending on their monomeric composition and chemical structure. More than 150 different monomers produced by bacteria from different carbon sources have been described up until now (Steinbüchel and Valentin. 1995. FEMS Microbiol. Lett. 128, 219-228), which gives an idea of the enormous diversity of PHAs that can be synthesized and the wide range of physical and mechanical properties they may have. Furthermore, these bioplastics are biodegradable and biocompatible, making them very suitable for medical applications, and 3-hydroxy acids obtained from the depolymerization thereof are compounds with high biotechnological potential.

Metabolic pathways have been described for PHA synthesis in several groups of bacteria (*Cupriavidus necator, Rhodospirillum rubrum, Pseudomonads).* One of the most widely studied groups are species from the genus *Pseudomonas* which are capable of accumulating PHA from fatty acids obtained from *de novo* synthesis and β-oxidation pathways (Rhem. 2010. Nature 8, 578-592; Koller et al. 2010. Food Technol. Biotechnol. 48, 255-269). Genes involved in PHAmcl biosynthesis have been characterized in several Pseudomonas species, and in all of them the genes are grouped in the *pha* cluster which is highly conserved between strains. The cluster is formed by two genes encoding PHA synthases (*phaC1* and *phaC2*), the *phaZ* gene encoding a depolymerase responsible for PHA mobilization and the *phaD* gene encoding a transcriptional regulator. Furthermore, there are other two genes in the cluster, i.e., *phaF* and *phaI,* which encode phasins.

Despite the enormous variability of PHAs which can be synthesized by microorganisms, only a few are produced on an industrial scale to be put on the market due to high production costs. PHA production involves steps of fermentation, separating the biomass from the culture broth, drying the biomass and extracting, purifying and drying the PHA (Queiroz et al. 2009. Polymer Reviews. 49,65-78; Shen et al. 2010. Biofuels, Bioprod. Bioref. 4, 25-40). Isolation of super-producing PHA strains, the increase in the substrate-PHA conversion rate, the design of a good fermentation process or the optimization of extraction and purification processes are key factors for achieving an industrially profitable PHA production process.

### Brief Description of the Invention

The objective of the present invention is to resolve production of plastic polymers of a microbial origin (polyhydroxyalkanoates) and monomers forming them (3-hydroxy acids) from the strategic, economic and technical viewpoints.

The authors of the present invention have developed a method for the production of plastic polymers of a microbial origin (bioplastics) and of monomers forming same. The method is based on the use of a new microorganism which is a super-producer and naturally capable of: a) metabolizing different carbon sources including aromatic derivatives, b) growing at high cellular concentrations, and c) producing a large amount of polyhydroxyalkanoates or 3-hydroxy acids. Both production processes reach high yields in a fast and simple manner and at a low production cost.

In a first aspect, the invention relates to a microorganism of the *Pseudomonas putida* species deposited in the Spanish Type Culture Collection (CECT) with accession number CECT 8092. Within said first aspect, the invention also relates to a microorganism of the *Pseudomonas putida* CECT 8092 species having a deletion in the *fadB* and *fadA* genes, and finally to a microorganism of the *Pseudomonas putida* CECT 8092 species having a deletion in the *fadB* and *fadA* genes and containing at least one additional copy of the *phaZ* gene itself, or in other words, having a higher gene dosage of the *phaZ* gene itself.

In a second aspect, the invention relates to a biologically pure culture of each of the microorganisms referred to in the preceding paragraph.

In a third aspect, the invention relates to a method for obtaining bioplastics comprising the following steps:
a. culturing a microorganism selected from *Pseudomonas putida* CECT 8092 and *Pseudomonas putida* CECT 8092Δ*fadBA* in the presence of at least one carbon source including a carboxylic acid, and of at least one nitrogen source,
b. separating the microbial biomass from the culture broth,
c. extracting the bioplastic from the microbial biomass obtained in the preceding step, and optionally,
d. purifying the bioplastic.

In a fourth aspect, the invention relates to a method for obtaining 3-hydroxy acids comprising the following steps:
a. culturing *Pseudomonas putida* CECT 8092Δ*fadBA* containing at least one additional copy of the *phaZ* gene itself in the presence of at least one carbon source including an arylcarboxylic acid, and of at least one nitrogen source,
b. separating the microbial biomass from the culture broth, and
c. extracting 3-hydroxy acids from the culture broth obtained in the preceding step.

In an additional aspect, the invention relates to the use of a microorganism of the *Pseudomonas putida* species deposited in the Spanish Type Culture Collection (CECT) with accession number CECT 8092 and the use of said microorganism having a deletion in the *fadB* and *fadA* genes in obtaining biopolymers.

In another additional aspect, the invention relates to the use of the preceding microorganism having a deletion and containing a higher gene dosage of the *phaZ* gene itself in obtaining 3-hydroxy acids.

In another additional aspect, the invention relates to a polynucleotide having the nucleotide sequence shown in SEQ ID NO 5.

Finally, in a final aspect, the invention relates to the use of the *phaZ* gene itself for increasing the yield in obtaining 3-hydroxy acids using a microorganism of the *Pseudomonas putida* CECT 8092 species and using a *Pseudomonas putida* CECT 8092 microorganism having a deletion in the *fadB* and *fadA* genes.

### Detailed Description of the Invention

### Pseudomonas putida CECT 8092

The first aspect of the invention relates to a microorganism of the *Pseudomonas putida* species deposited in the Spanish Type Culture Collection (CECT) with accession number CECT 8092. This super-producing *Pseudomonas putida* strain was selected by applying standard methods (Gómez et al. 1996. Appl. Microbiol. Biotecnol. 45, 785-791) on a sample containing soil, plant remains, etc. Said strain was identified as *Pseudomonas putida* by means of sequencing the *rpoB* gene (SEQ ID NO 1) and the 16S ribosomal DNA subunit (SEQ ID NO 2), showing differences with other sequences of this species deposited in the consulted databases. The microorganism has been deposited in the Spanish Type Culture Collection as *Pseudomonas putida* CECT 8092.

The present *Pseudomonas putida* strain is capable of metabolizing different carbon sources including aromatic derivatives (n-phenylalkanoic acids), growing at high cellular concentrations (OD600nm greater than 250 and biomass densities of 60 g/l), and finally, producing a large amount of polyhydroxyalkanoates and 3-hydroxy acids in a fast and simple manner and at a low production cost. As demonstrated in the examples included in the text of the present application (Example 1 and Table 1 in Example 4), this strain can accumulate over 50% of its dry weight in the form of polyhydroxyalkanoates, with a yield of 0.5 in the conversion of the acid used into PHA.

In the present invention, "bioplastics" is understood as those plastics obtained from natural sources (microbial origin) or (completely or partially) renewable sources, as well as biodegradable plastics which can decompose in naturally occurring conditions by means of the enzymatic action of microorganisms such as bacteria, fungi and algae. Particularly, the polyhydroxyalkanoates obtained by means of the microorganisms of the present invention are bioplastics. Generally, "plastics" are synthetic materials obtained by means of polymerization phenomena or semi-natural multiplication of carbon atoms into long molecular chains of organic compounds derived from petroleum and other natural substances.

### Pseudomonas putida CECT 8092ΔfadBA

Within the first aspect and in a particular embodiment, the invention relates to the microorganism *Pseudomonas putida* CECT 8092Δ*fadBA.* This microorganism corresponds to *Pseudomonas putida* CECT 8092 having a deletion of the *fadA* gene (SEQ ID NO 3) and *fadB* gene (SEQ ID NO 4) involved in fatty acid metabolism. For this reason, the strain or microorganism having a deletion has increased production of polyhydroxyalkanoates (polymers or bioplastics). Therefore, as shown in the examples of the present application (see Table 1), the production of polyhydroxyalkanoates (PHAs) produced by the microorganism *Pseudomonas putida* CECT 8092Δ*fadBA* is between 5 and 32% greater than that obtained by the parent strain or microorganism.

### PhaZ gene

Also within the first aspect and in another particular embodiment, the invention relates to another new microorganism corresponding to *Pseudomonas putida* CECT 8092Δ*fadBA* containing a higher gene dosage of the *phaZ* gene itself (SEQ ID NO 5), i.e., containing at least one additional copy of the *phaZ* gene itself. In the context of the present invention, "at least one additional copy" is understood as the presence of one or more additional copies, preferably between 1 and 20, more preferably between 5 and 20 additional copies. This gene encodes a depolymerase the expression of which prevents intracellular formation and accumulation of polyhydroxyalkanoates, favoring, in contrast, the release of 3-hydroxy acids into the culture medium (Ren et al. 2005, Biomacromolecules 6:2290-2298; Prieto et al. 2007, Ramos JL and Filloux A (Eds.) Pseudomonas, 5. Springer, Amsterdam, 397-428; Sandoval et al. 2005, Appl. Microbiol. Biotechnol 67, 97-105). The presence of several copies of this gene in said microorganism results in an increase in 3-hydroxycarboxylic acid production yield. In a preferred embodiment of the invention, the number of additional copies of the *phaZ* gene itself present in the microorganism is from 5 to 20 copies. Therefore, as shown in the examples of the present application, the yield in the conversion of an n-phenylalkylcarboxylic acid into 3-OH-phenylalkylcarboxylic acid may exceed 75%.

In a second aspect, the invention relates to a biologically pure culture of each of the new microorganisms described in the present invention. In the present invention, "biologically pure culture" is understood as that culture in which the microorganism of the invention is at a proportion equal to or greater than 95% with respect to the remaining microorganisms present in the culture. In the text of the present application, biologically pure cultures of the microorganisms of the invention are illustrated with examples.

### Method for obtaining bioplastics

In a third aspect, the invention relates to a method for obtaining bioplastics, particularly polyhydroxyalkanoates, comprising the following steps:
a. culturing a microorganism selected from *Pseudomonas putida* CECT 8092 and *Pseudomonas putida* CECT 8092Δ*fadBA* in the presence of at least one carbon source including a carboxylic acid, and of at least one nitrogen source,
b. separating the microbial biomass from the culture broth,
c. extracting the bioplastic from the microbial biomass obtained in the preceding step, and optionally,
d. purifying the bioplastic

Culturing the microorganism in step a) involves growing the producer microorganism, either *Pseudomonas putida* CECT 8092 or *Pseudomonas putida* CECT 8092Δ*fadBA,* preferably in flasks or bioreactors until reaching a polyhydroxyalkanoate content that is preferably between 20% and 70% of the dry weight.

The microorganism is grown in culture media in which there is at least one carbon source including a carboxylic acid, and at least one nitrogen source.

The carbon source includes a carboxylic acid. The carboxylic acid has a straight or branched alkyl chain which can have between 3 and 13, preferably between 5 and 11 carbon atoms, and it can have at least one hydrogen substituted with an OH group, an ester group or an amino group. The chain can also contain thiol groups, halogen groups and have one or more unsaturations. Said chain can also have aryl substituents (arylcarboxylic acid), particularly in position n of the chain (n-arylcarboxylic acid), i.e., in the position opposite the carboxyl group.

In the present invention, an "aryl" group is understood as an aromatic group having between 6 and 18, more preferably 6 or 10 carbon atoms, comprising 1, 2 or 3 aromatic nuclei bound by a carbon-carbon bond or fused to one another. Illustrative examples of aryl groups include phenyl, naphthyl, diphenyl, indenyl, etc.

The carboxylic acid preferably has a straight chain. In a particular embodiment, the carboxylic acid is an arylcarboxylic acid, preferably n-arylcarboxylic acid, or a fatty acid.

In a preferred embodiment, the carbon source is a carboxylic acid having between 4 and 14 carbon atoms, more preferably between 6 and 12 carbon atoms; or an aryl-carboxylic acid, preferably n-arylcarboxylic acid, having between 10 and 32 carbon atoms, more preferably n-phenyl-carboxylic acid. In a preferred embodiment, the carbon source additionally includes glucose, lactose, glycerol, molasses, mannose, fructose, acetate or combinations thereof, particularly glucose or glycerol or combinations thereof.

In another preferred embodiment of the invention, the carbon source used is a mixture of glycerol and preferably a straight-chain carboxylic acid, containing between 6 and 12 carbon atoms. Glycerol is used in an amount between 0.1% (w/v) and 10% (w/v), preferably between 2% (w/v) and 6% (w/v), and more preferably between 3% (w/v) and 5% (w/v). In another preferred embodiment of the invention, the carbon source used is a mixture of glucose and preferably a straight-chain carboxylic acid, containing between 6 and 12 carbon atoms. Glucose is used in an amount between 0.1% (w/v) and 10% (w/v), preferably between 2% (w/v) and 6% (w/v), and more preferably between 3% (w/v) and 5% (w/v).

In another embodiment of the invention, the carboxylic acid used as a carbon source is selected from hexanoic acid, heptanoic acid, octanoic acid, nonanoic acid and decanoic acid. These acids are used at a concentration between 5 mM and 500 mM, preferably between 10 and 400 mM, and more preferably between 20 and 250 mM.

In another embodiment of the invention, the n-phenylcarboxylic acid used as a carbon source is selected from 6-phenylhexanoic acid, 7-phenylheptanoic acid and 8-phenyloctanoic acid. These acids are used at a concentration between 5 mM and 200 mM, preferably between 10 and 150 mM, and more preferably between 15 and 100 mM.

In a particular embodiment, the microorganism is initially fed using glycerol and/or glucose as the carbon source, to subsequently use a carboxylic acid as the carbon source.

The carbon source is normally in excess in the ratio of mass to nitrogen source present in the same medium.

The nitrogen source can be selected from the group consisting of yeast extract, peptone, corn steep liquor, urea, sodium glutamate and inorganic nitrogen sources, such as different ammonium salts (chloride, nitrate, sulfate), for example, and combinations thereof.

In one embodiment of the invention, the microorganisms are cultured for 2 to 5 days at a temperature between 18°C and 37°C, preferably between 25°C and 35°C, and more preferably between 28°C and 32°C, with constant stirring.

Once the microorganisms have been cultured, preferably until reaching the maximum intracellular amount of polyhydroxyalkanoates, the microbial or cellular biomass is separated from the culture broth. This separation is performed by means of any of the methods commonly used for this purpose, such as, for example, filtration, microfiltration, centrifugation, or combinations thereof, among others. In a preferred embodiment, after performing the step of separating the microbial biomass from the culture broth (step b), and before extracting the bioplastic from the biomass (step c), the biomass is dried in a stove or oven, preferably at 50-70°C and for 48-72 hours, or the separated biomass is lyophilized.

The third step of the method (step c) comprises extracting the bioplastic from the microbial or cellular biomass obtained in the preceding step of the method. In a particular embodiment, said extraction is performed by means of a solid-liquid extraction process. Any water-immiscible organic solvent can be used as solvent, such as, for example, n-hexane, toluene, acetone, ethyl acetate or dimethoxymethane, among others. Known examples of extracting polyhydroxyalkanoates from a biomass with organic solvents are described in patent documents such as US 4,310,684 and US 4,705,604, particularly extracting PHB (poly-hydroxybutyrate) with chlorinated solvents; US 4,968,611 describes the use of diols, butyrolactone or di- or tricarboxylic acid esters for extracting PHB and copolymers thereof; US 5,213,976 describes extracting PHB with methylene chloride followed by precipitation with water. Patent documents WO97/15681 and WO93/11656 describe extracting poly-3-hydroxyoctanoate from *Pseudomonas oleovorans* with acetone; WO2005/052175 describes a process for extracting PHB and PHBV (poly-hydroxybutyrate-co-hydroxyvalerate) with different types of solvents. Finally, EP1781721 describes extracting PHB-co-PHH copolymer by means of using acetone, methyl-ethyl ketones, or mixtures thereof.

Depending on their degree of purity and subsequent use, the polymers extracted in step c) can be subjected to a purification process (step d). This optional last step of the method is generally based on precipitating the impurities included in the product resulting from step c) and can be performed by means of adding a precipitating agent in which the polyhydroxyalkanoates are not soluble, such as, for example, water or different types of alcohols, such as methanol, ethanol, isopropanol or n-butanol (WO00/68409, WO2005/052175), by means of adding hydrogen peroxide and chelating agents (US 5,691,174), or by means of adding ozone (WO99/51760).

The polyhydroxyalkanoates (bioplastics) obtained using the method of the invention are copolymers formed from derivatives of the carboxylic acid used as a carbon source. Therefore, if the carbon source is octanoic acid, copolymers formed by 3-hydroxyoctanoate (80-90%) and 3-hydroxyhexanoate (20-10%) will be obtained. Said polyhydroxyalkanoates (bioplastics) can be used as raw material for the production of different products such as, for example, containers, packaging films, biomedical devices, personal hygiene products, bags, etc. (Philip et al. 2007. J. Chem. Technol. Biotechnol., 82, 233; Mikova et al. 2006. Chem. Listy, 100, 1075; Chen et al. 2005. Biomaterials, 67, 592; Chen GQ. 2009. Chem. Soc. Rev., 38, 2434-2446).

### Method for obtaining 3-hydroxy acids

In a fourth aspect, the invention relates to a method for obtaining 3-hydroxy acids comprising the following steps:
a. culturing *Pseudomonas putida* CECT 8092Δ*fadBA* containing at least one additional copy of the *phaZ* gene itself in the presence of at least one carbon source including an arylcarboxylic acid, and of at least one nitrogen source,
b. separating the microbial biomass from the culture broth, and
c. extracting 3-hydroxy acids from the culture broth of the preceding step.

In a preferred embodiment of the invention, the number of additional copies of the *phaZ* gene itself present in the microorganism having a deletion is from 1 to 20 copies, more preferably from 5 to 20 copies.

The microorganism is cultured in a similar manner as in step a) of the method for obtaining bioplastics described above. However, the carbon source in this method necessarily includes an arylcarboxylic acid, preferably an n-arylcarboxylic acid, having preferably between 10 and 32 carbon atoms, more preferably n-phenyl-carboxylic acid. The nitrogen source is the same as that used in the method for obtaining bioplastics described above.

In one embodiment of the invention, the microorganisms are cultured for 2 to 5 days at a temperature between 18°C and 37°C, preferably between 25°C and 35°C, and more preferably between 28°C and 32°C, with constant stirring.

Preferably once maximum production of 3-hydroxy acids is reached, the microbial or cellular biomass is separated from the culture broth in step b by means of any of the methods commonly used for this purpose, such as, for example, filtration, microfiltration, centrifugation or combinations thereof, among others.

The culture broth obtained in step b is then subjected to extraction to recover 3-hydroxy acids (step c). In a particular embodiment, this extraction consists of extracting said culture broth (aqueous phase) with an organic solvent. Organic solvents suitable for this extraction are, among others, diethyl-ether, methyl-tert-butyl-ether, methylene chloride and ethyl acetate.

The 3-hydroxy acids obtained in the method described in the present invention are those derived from the alkylcarboxylic acid used as a carbon source. Therefore, if the carbon source is a phenylhexanoic acid, the product obtained will be 3-hydroxyhexanoic acid. The 3-hydroxy acids obtained can be used as precursors in the pharmaceutical industry for synthesizing compounds such as antibiotics, vitamins or pheromones (Chen G.Q. 2009, Chem. Soc. Rev. 38, 2434-2446).

In an additional aspect, the invention relates to the use of the following microorganisms in obtaining biopolymers, particularly polyhydroxyalkanoates:
- *Pseudomonas putida* CECT 8092 and
- *Pseudomonas putida* CECT 8092 having a deletion in *fadB* and *fadA* genes.

In another additional aspect, the invention relates to the use of the following microorganism in obtaining 3-hydroxy acids:
- *Pseudomonas putida* CECT 8092 having a deletion in *fadB* and *fadA* genes containing
at least one additional copy of the *phaZ* gene itself. In the present application, the expression "more than one copy of the *phaZ* gene itself" is understood as the microorganism containing a higher gene dosage of the *phaZ* gene itself, i.e., the polynucleotide having sequence ID SEQ NO 5. Said additional copy/copies of the *phaZ* gene itself are introduced in said microorganism following standard methods (Sambrook J. and Russell D. W. 2001., Cold Spring Harbor Laboratory Press, Cold Spring Harbor, NY), as illustrated in Example 5 of the application. The number of additional copies of the *phaZ* gene itself is preferably from 1 to 20, more preferably from 5 to 20 additional copies.

In another additional aspect, the invention relates to a polynucleotide having the nucleotide sequence shown in SEQ ID NO 5. This polynucleotide corresponds to the *phaZ* gene of the microorganism of the invention *Pseudomonas putida* CECT 8092.

Finally, in a final aspect, the invention relates to the use of a polynucleotide having the nucleotide sequence shown in SEQ ID NO 5 for increasing yield in obtaining 3-hydroxy acids using a microorganism of the *Pseudomonas putida* CECT 8092 species having a deletion in the *fadB* and *fadA* genes.

### Examples

### 1. Production of PHA

PHA synthesis in 30-liter bioreactors with the *P. putida* CECT 8092 strain was performed in an M9 medium supplemented with a trace element solution (Abril et al. 1989, J. Bacteriol. 171, 6782-6790) with glycerol (30 g/L) as the initial carbon source. The culture was grown to an OD600nm of 20, and an octanoic acid feeding phase was started after that time. After culturing for 60 hours, 60 g/L of biomass with a PHA content exceeding 50% the dry weight thereof were obtained.

Once the cellular biomass was separated from the culture broth by means of microfiltration, it was dried at 65°C for 3-5 days. The dry biomass was subjected to an extraction process with ethyl acetate. The mixture was kept in the Shoxlet apparatus at 35-40°C for 20 hours. Once the extraction ended, the solvent was evaporated and the obtained PHA (copolymer formed by about 90% 3-hydroxyoctanoic and 10% 3-hydroxyhexanoic acid) was purified with isopropanol. The final amount of purified PHA obtained was 625 g, which is a yield of 47.60% in the conversion of octanoic acid into PHA.

### 2. P. putida CECT8092 ΔfadBA strain construct

Once the sequence of the *fadA* gene (SEQ ID NO 3) and *fadB* gene (SEQ ID NO 4) of *P. putida* CECT 8092 strain was obtained, the construct to be used for deletion was designed. To that end, two fragments located at the ends of the *fadBA* genes were amplified by PCR. One of said fragments, i.e., the one having 665 bps, is located at the 5' end of the *fadB* gene, and the other fragment, i.e., the one having 667 bps, is located at the 3' end of the *fadA* gene. These two fragments were ligated, and the resulting construct was cloned into the pGEM-Teasy plasmid, from which the fragments were released and cloned into pJQ200KS (plasmid containing the gentamicin resistance marker and the *sacB* gene encoding sucrase). That construct was transferred to *E. coli* and then to *P. putida* CECT 8092 by means of conjugation.

To obtain a mutant having a deletion by means of double recombination, first a selection was made in an LB medium supplemented with ampicillin and gentamicin. The transformants selected in this medium were then grown in LB medium supplemented with ampicillin and 10% sucrose.

The transconjugants were analyzed by means of PCR using the genomic DNA thereof. To that end, specific oligonucleotides corresponding to the outer regions of the fragments cloned into the pJQ200KS vector were used.

### 3. Production of PHA from P. putida CECT8092 ΔfadBA

*P. putida* CECT 8092 and *P. putida* CECT 8092 Δ*fadBA* were grown in respective flasks in a defined minimal medium (Martínez-Blanco H. 1990, J. Biol. Chem. 265, 7084-7090), the composition of which in g l⁻¹ is: KPO₄H₂, 13.6; (NH₄)₂SO₄, 2.0; MgSO₄ 7H₂O, 0.25; FeSO₄ 7H₂O, 0.0005. A mixture consisting of octanoic acid at a final concentration of 30 mM and 1% glucose (w/v) was used as the carbon source. A cellular suspension of the corresponding strain was inoculated into 500 mL of medium. The flasks were incubated at 30°C and 250 rpm for the required time. When the culture reached stationary phase, the bacteria were collected by centrifugation, and after eliminating the supernatant, they were frozen at -80°C. The bacteria were then lyophilized and this material was used for extracting PHAs as described in Example 1.

The results obtained in the different media are shown in Table 1. The final amount of PHA obtained using the *P. putida* CECT 8092 Δ*fadBA* strain was 53.1% the dry weight thereof, i.e., about 5% more than that obtained with the parent strain.

### 4. Production of PHA containing aromatic residues

*P. putida* CECT 8092 and *P. putida* CECT 8092 Δ*fadBA* were grown in respective flasks in a defined minimal medium described in Example 3. A mixture consisting of different n-phenylalkanoic acids (6-phenylhexanoic acid, 7-phenylheptanoic acid and 8-phenyloctanoic acid) at a final concentration of 15 mM and 1% glucose (w/v) were used as carbon sources. A cellular suspension of the corresponding strain was inoculated into 500 mL of medium. The flasks were incubated at 30°C and 250 rpm for the required time. When the culture reached stationary phase, the bacteria were collected by centrifugation, and after eliminating the supernatant, they were frozen at -80°C. The bacteria were then lyophilized and this material was used for extracting PHAs following the method described in Example 1. The results obtained in the different media are shown in Table 1 as the % of dry weight of the microorganism.

**Table 1. Production of PHA in the strains in a defined medium supplemented with different fatty acids**

| | MM + 1% glucose | | | |
|---|---|---|---|---|
| | + 30 mM octanoic acid | 15 mM 6-Φ-hexanoic acid | 15 mM 7-Φ-heptanoic acid | 15 mM 8-Φ-octanoic acid |
| *P. putida* CECT 8092 | 47.60% | 17.3% | 36.2% | 40.6% |
| *P. putida* CECT 8092 Δ*fadBA* | 53.1% | 49.7% | 55.9% | 61.3% |

Depending on the precursor used, the final amount of PHA produced by the *P. putida* CECT 8092 Δ*fadBA* strain was between 19% and 32% more than that obtained with the parent strain.

### 5. Production of 3-hydroxy acids

The *phaZ* gene of *P. putida* CECT 8092 was amplified by means of PCR (SEQ ID NO 5) and cloned into the pBBR1-MCS3 (Tc^{R}) plasmid.

The construct was transferred to E. *coli* DH10B by means of transformation and successful incorporation of the plasmid was checked by means of sequencing the *phaZ* gene. The plasmid was then transferred to the *P. putida* CECT 8092 Δ*fadBA* strain by means of conjugation. To check the success of the construct, plasmid was extracted from the transconjugants and was used for transforming E. *coli* DH10B. Plasmid was then extracted from the *E. coli* strain, and the *phaZ* gene was amplified by means of PCR. Sequencing the obtained fragment confirmed that the *Pseudomonas* strain contained the desired construct.

To analyze the production of 3-hydroxy acids by the P. *putida* CECT 8092 Δ*fad*BA strain containing at least one additional copy of the *phaZ* gene itself, this strain was cultured in the medium described in Example 3, but using glycerol instead of glucose and 6-phenylhexanoic acid instead of octanoic acid. The flasks were incubated at 30°C and 250 rpm for 72 hours. The cells were collected by centrifugation and the culture broth was filtered through a 0.2 µm filter. The 3-OH-monomers were obtained by means of a liquid/liquid extraction with organic solvents, such as methylene chloride, diethyl ether or ethyl acetate (Sandoval et al. 2005, Appl. Microbiol. Biotechnol, 67, 97-105). The obtained organic phase (ethyl acetate extract) was dried with anhydrous sodium sulfate, and an aliquot was analyzed by means of HPLC/Mass spectrometry and NMR, confirming that the compounds contained therein consisted of a mixture formed by 3-hydroxy-6-phenylhexanoic acid (more than 90%), traces of free acids and 3-hydroxy-4-phenylbutanoic acid, as well as esters of both acids. The rest of the obtained extract was evaporated to dryness under a nitrogen stream and then dried in a lyophilizer for 12 hours.

The components of this extract were separated and characterized by means of semi-preparative HPLC (9.4 X 250 mm, 5 um Zorbax RX-C8 column with 5-100% gradient of ACN/water with 0.1% TFA, flow rate of 3.6 ml/min, UV detection at 210 and 340 nm). Analysis of the major fraction of the extract (more than 90% of the total) by means of CDCl₃ NMR confirmed the structure corresponding to 3-hydroxy-phenylhexanoic acid. The total yield in conversion from 6-phenylhexanoic acid was 75%.

## Claims

1. A microorganism of the *Pseudomonas putida* species deposited in the Spanish Type Culture Collection (CECT) with accession number CECT 8092.

2. The microorganism according to claim 1 having a deletion in the *fadB* and *fadA* genes.

3. The microorganism according to claim 2 containing at least one additional copy of the *phaZ* gene itself.

4. A biologically pure culture of a microorganism according to any of claims 1 to 3.

5. A method for obtaining bioplastics comprising the following steps:
a. culturing a microorganism according to any of claims 1 or 2 in the presence of at least one carbon source including a carboxylic acid and of at least one nitrogen source,
b. separating the microbial biomass from the culture broth,
c. extracting the bioplastic from the microbial biomass obtained in the preceding step, and optionally,
d. purifying the bioplastic.

6. The method according to claim 5, wherein the carboxylic acid has a straight chain.

7. The method according to any of claims 5 and 6, wherein the carboxylic acid is an arylcarboxylic acid, preferably n-arylcarboxylic acid or a fatty acid.

8. The method according to any of claims 5 to 7, wherein the carboxylic acid without aromatic substitution has between 4 and 14, preferably between 6 and 12, carbon atoms, and the arylcarboxylic acid has between 10 and 32 carbon atoms and is preferably n-phenylcarboxylic acid.

9. The method according to any of claims 5 to 8, wherein the carbon source also includes glucose, lactose, glycerol, molasses, mannose, fructose, acetate, or combinations thereof.

10. The method according to any of claims 5 to 9, wherein the obtained polymers contain 3-hydroxy acids with aliphatic chains having a length of between 3 and 13, preferably of between 5 and 11 carbon atoms.

11. The method according to any of claims 5 to 9, wherein the obtained polymers contain at least one aromatic residue, preferably phenyl.

12. A method for obtaining 3-hydroxy acids comprising the following steps:
a. culturing the microorganism according to claim 3 in the presence of at least one carbon source including an arylcarboxylic acid and of at least one nitrogen source,
b. separating the microbial biomass from the culture broth, and
c. extracting 3-hydroxy acids from the culture broth of the preceding step.

13. The method according to claim 12, wherein the arylcarboxylic acid has a straight alkyl chain.

14. The method according to claim 13, wherein the arylcarboxylic acid is an n-arylcarboxylic acid.

15. The method according to claim 14, wherein the n-arylcarboxylic acid has between 10 and 32 carbon atoms, it is preferably an n-phenylcarboxylic acid.

16. The method according to any of claims 12 to 15, wherein the carbon source also includes glucose, lactose, glycerol, molasses, mannose, fructose, acetate or combinations thereof.

17. The method according to any of claims 12 to 16, wherein the obtained hydroxy acids contain at least one aromatic residue, preferably phenyl, and an aliphatic chain having a length of between 3 and 10, preferably of between 4 and 8 carbon atoms.

18. Use of a microorganism according to any of claims 1 or 2 in obtaining biopolymers.

19. Use of a microorganism according to claim 3 in obtaining 3-hydroxy acids.

20. A polynucleotide having the nucleotide sequence shown in SEQ ID NO: 5.

21. Use of the polynucleotide according to claim 20 to increase the yield in obtaining 3-hydroxy acids using a microorganism according to claim 2.


SEQUENCE LISTING

<110>  NEOL BIOSOLUTIONS, S.A.

<120>  PRODUCTION OF BIOPLASTICS

<130>  P8398EPPC

<140>  EP13829939.1
<141>  2015-03-06

<150> P201231305
<151> 2012-08-14

<160>  5

<170>  PatentIn version 3.5

<210>  1
<211>  1229
<212>  DNA
<213>  Pseudomonas putida, rpoB gene

<400>  1
ggccgagaac cagttccgcg ttggcctggt gcgtgtcgag cgcgcggtca aggaacgcct      60

gtcgatggcg gaaagcgaag gcctgatgcc gcaagacctg atcaacgcca agccggtagc     120

ggcggcggtg aaagagttct ttggctccag ccagctgtcc cagttcatgg accagaacaa     180

ccctctctcg gagattaccc acaagcgccg cgtctctgca ctcggccctg gcggtctgac     240

ccgtgagcgt gccggcttcg aagtccgtga cgtacacccg acccactatg gccgtgtgtg     300

cccgatcgag acccctgaag gtccgaacat cggtctgatc aactccttgg cggcctacgc     360

ccgcaccaac cagtacggct tcctggaaag cccgtaccgc gtggttaagg aaggcgttgt     420

aagtgacgac atcgtgttcc tgtcggcaat cgaagaggcg gatcacgtca tcgcacaggc     480

ttcggccgcg atgaacgaca agaagcaact gatcgatgag ctggtagcag ttcgtcacct     540

gaacgaattc accgtcaagg cgccggaaga cgtcaccctg atggacgttt cgccgaagca     600

ggttgtttcc gttgcagcgt cgctgattcc gttcctcgag cacgacgacg ccaaccgtgc     660

gttgatgggt tcgaacatgc agcgtcaggc tgtaccaacc ctgcgtgccg acaagccgct     720

ggtaggtacc ggcatggagc gcaacgttgc ccgtgactcc ggtgtctgtg tggttgctcg     780

ccgcggtggt gtgatcgact cggtcgacgc tagccgtatc gttgttcgcg ttgccgacga     840

cgaagtggaa accggcgaag caggtgtgga tatctacaac ctgaccaagt acacccgttc     900

gaaccagaac acctgcatca accagcgtcc gctggtgagc aaaggtgaca aggttcagcg     960

tggtgacatc atggccgacg gcccgtccac cgacatgggt gagctggctc tgggtcagaa    1020

catgcgcatc gcgttcatgg cgtggaacgg cttcaacttc gaagactcca tctgcctgtc    1080

cgagcgtgtg gttcaggaag atcgcttcac caccatccac attcaggaac tgacctgtgt    1140

ggcgcgtgac accaagctcg gcccagagga aatcactgcg gacatcccga acgtgggtga    1200

agccgcactg aacaagctgg acgaagccg                                    1229

<210>  2
<211>  1460
<212>  DNA
<213>  Pseudomonas putida, 16S ribosomal RNA

<400>  2
attgaacgct ggcggcaggc ctaacacatg caagtcgagc ggatgacggg agcttgctcc      60

ttgattcagc ggcggacggg tgagtaatgc ctaggaatct gcctggtagt gggggacaac     120

gtttcgaaag gaacgctaat accgcatacg tcctacggga gaaagcaggg gaccttcggg     180

ccttgcgcta tcagatgagc ctaggtcgga ttagctagtt ggtggggtaa tggctcacca     240

aggcgacgat ccgtaactgg tctgagagga tgatcagtca cactggaact gagacacggt     300

ccagactcct acgggaggca gcagtgggga atattggaca atgggcgaaa gcctgatcca     360

gccatgccgc gtgtgtgaag aaggtcttcg gattgtaaag cactttaagt tgggaggaag     420

ggcagtaagc taataccttg ctgttttgac gttaccgaca gaataagcac cggctaactc     480

tgtgccagca gccgcggtaa tacagagggt gcaagcgtta atcggaatta ctgggcgtaa     540

agcgcgcgta ggtggtttgt taagttggat gtgaaagccc cgggctcaac ctgggaactg     600

catccaaaac tggcaagcta gagtacggta gagggtggtg gaatttcctg tgtagcggtg     660

aaatgcgtag atataggaag gaacaccagt ggcgaaggcg accacctgga ctgatactga     720

cactgaggtg cgaaagcgtg gggagcgaac aggattagat accctggtag tccacgccgt     780

aaacgatgtc aactagccgt tggaatcctt gagattttag tggcgcagct aacgcattaa     840

gttgaccgcc tggggagtac ggccgcaagg ttaaaactca aatgaattga cgggggcccg     900

cacaagcggt ggagcatgtg gtttaattcg aagcaacgcg aagaacctta ccaggccttg     960

acatgcagag aactttccag agatggattg gtgccttcgg gaactctgac acaggtgctg    1020

catggctgtc gtcagctcgt gtcgtgagat gttgggttaa gtcccgtaac gagcgcaacc    1080

cttgtcctta gttaccagca cgttatggtg ggcactctaa ggagactgcc ggtgacaaac    1140

cggaggaagg tggggatgac gtcaagtcat catggccctt acggcctggg ctacacacgt    1200

gctacaatgg tcggtacaga gggttgccaa gccgcgaggt ggagctaatc tcacaaaacc    1260

gatcgtagtc cggatcgcag tctgcaactc gactgcgtga agtcggaatc gctagtaatc    1320

gcgaatcaga atgtcgcggt gaatacgttc ccgggccttg tacacaccgc ccgtcacacc    1380

atgggagtgg gttgcaccag aagtagctag tctaaccttc gggaggacgg ttaccacggt    1440

gtgattcatg actggggtga                                                1460

<210>  3
<211>  1176

<212>  DNA
<213>  Pseudomonas putida, fadA gene

<400>  3
atgagcctga atccaagaga cgtggtgatt gtcgacttcg gtcgcacgcc aatgggccgc      60

tccaagggtg gcatgcaccg caacacccgc gccgaagaca tgtcggcgca cctgatcagc     120

aagctgctgg aacgcaacgg caaggttgac ccgaaagaag tcgaggacgt gatctggggc     180

tgcgtcaacc agaccctgga gcagggctgg aacatcgccc gcatggcctc gctgatgacc     240

cagatcccgc acacttccgc ggcgcagacc gtcagccgcc tgtgcggctc gtccatgagc     300

gcgctgcaca cggccgccca ggcgatcatg accggtaacg gtgatgtgtt cgtggtcggt     360

ggcgtggagc acatgggcca cgtcagcatg atgcatggcg tagaccccaa cccgcacctg     420

tccttgcatg ccgccaaggc ttccgggatg atgggcctga ctgcagaaat gctcggcaag     480

atgcacggca tcacccgtga gcagcaggac ctgttcggct tgcgttcgca ccagctggcc     540

cacaaggcca cggtcgaagg caagttcaag gacgagatca tcccgatgca gggctacgac     600

gagaacggct tcctgaaggt gttcgatttc gacgaaacca ttcgcccgga aaccaccctc     660

gaaggcctgg catcgctgaa gcctgcgttc aacccgaaag gcggtacggt cacggccggt     720

acctcgtcgc agatcaccga cggcgcctcg tgcatgatcg tcatgtccgg tcagcgtgcc     780

atggacctcg gtatccagcc attggcggtg atccgttcga tggcagtggc cggtgtcgac     840

ccggcaatca tgggctacgg cccggtgcca tcgacccaga aagccctcaa gcgtgcgggc     900

ttgaccatgg ccgatatcga cttcatcgag ctcaacgaag ccttcgctgc acaggccctg     960

cccgtgctga aagacttgaa agtgctcgac aagatggatg agaaggttaa cctgcacggc    1020

ggcgccattg ctttgggcca cccgtttggt tgctccgggg cgcggatttc cggcaccctg    1080

ctcaacgtca tgaagcaaaa tggcggtacg ctgggtgttg cgaccatgtg cgtcggcctg    1140

ggccaaggta tcaccactgt ctttgaacgc gtctga                              1176

<210>  4
<211>  2148
<212>  DNA
<213>  Pseudomonas putida, fadB gene

<400>  4
atgatttacg aaggtaaagc catcacggtt aaggctcttg aaagtggcat cgtcgagctc      60

aagttcgacc tcaagggtga gtccgtcaac aagttcaacc gccttaccct gaacgagctg     120

cgccaggccg tcgatgccat ccaggccgat gcctcggtca aaggcgtgat cgtcagcagt     180

ggcaaggacg tgttcatcgt cggcgccgac atcaccgagt tcgtcgacaa cttcaagctg     240

cctgaggccg aactggtcgc cggcaacctg gaagccaatc gtatcttcaa cgccttcgaa     300

gacctcgaag tgccgaccgt tgccgccatc aacggcatcg cgctgggcgg cggcctggaa     360

atgtgcctgg cggccgacta ccgggtcatg tccaccagcg ccaggatcgg cctgccggaa 420

gtcaagctgg gtatctaccc gggctttggc ggtaccgtgc gcctgccgcg cctgatcggc 480

tcggacaacg ccatcgagtg gatcgccgcc ggcaaggaaa accgtgccga agacgccctg 540

aaagtggggg ccgtcgacgc ggtggtcgcc cctgagctgc tgctggccgg tgccctcgac 600

ctgatcaagc gtgccatcag tggcgagctg gactacaagg ccaagcgcca gccgaagctg 660

gaaaagctca agctcaatgc catcgagcag atgatggcct tcgagacggc caagggcttc 720

gtcgctggcc aggccggccc gaactaccca gccccggtcg aagcaatcaa gagcatccag 780

aaagccgcca acttcggtcg cgacaaggcc ctggaagttg aagccgcagg ctttgccaag 840

ctggccaaga cctcggtcgc cgagagcctg atcggcttgt tcctcaacga tcaggaactc 900

aagcgcaagg ccaaggcgca tgacgagatc gcccacgacg tgaagcaggc cgccgtgctc 960

ggcgccggca tcatgggcgg cggtatcgcc taccagtcgg cggtcaaagg tacgccgatc 1020

ctgatgaaag acatccgcga agaagccatt cagctgggtc tgaacgaggc ctccaagttg 1080

cttggcaacc gcgtcgagaa gggccgcctg accccagcca agatggccga ggccctcaac 1140

gccattcgac cgaccctgtc ctatggcgat tttgccaatg tcgacatcgt cgtcgaggca 1200

gtggtcgaga acccgaaggt caagcaagcg gtactggcgg aagtggaagg ccaggtgaag 1260

gacgatgcga tcctcgcttc caacacctct accatctcta tcaacctgct ggccaaggcg 1320

ctcaagcgcc cggaaaactt cgtcggcatg cacttcttca acccggtgca catgatgccg 1380

ctggtggaag tgatccgtgg tgagaagtcc agtgacgtgg cggtcgccac caccgtggcc 1440

tacgccaaga aaatgggcaa gaacccgatc gtggtcaacg actgcccggg ctttttggtc 1500

aaccgcgtgc tgttcccgta ctttggcggt tttgccaagc tggtcagcgc cggtgtcgac 1560

ttcgtgcgca tcgacaaggt catggagaag ttcggctggc cgatgggccc agcctacttg 1620

atggacgtgg tcggcatcga caccggccac cacggccgtg acgtcatggc cgaaggcttc 1680

ccggatcgca tgaaggacga gcgccgctcg gcagtcgacg cgttgtacga ggccaaccgc 1740

ctgggccaga agaacggtaa gggcttctac gcctacgaaa ccgacaagcg cggcaagccg 1800

aagaaggttt tcgatgccac cgtgctcgac gtgctcaaac cgatcgtgtt cgagcagcgc 1860

gaagtcactg acgaagacat catcaactgg atgatggtcc cgctgtgcct ggagaccgtg 1920

cgttgcctgg aagacggcat cgtcgaaacc gctgccgaag ccgacatggg cctggtctac 1980

ggcattggtt tccctccctt ccgcggtggt gcgctgcgtt acatcgactc gatcggtgtg 2040

gccgaattcg tcgccctggc cgatcagtat gccgacctgg ggccgctgta ccacccgacc 2100

gccaagctgc gtgaaatggc caagaacggc cagcgcttct tcaactga 2148

<210> 5
<211> 852

<212>  DNA
<213>  Pseudomonas putida, phaZ gene

<400>  5
atgccgcaac cctatatttt caggaccgtc gagctggacc accagtccat ccgcaccgct      60

gttcgccccg gcaaaccgca cctgacgccg ttgctgatct tcaacggcat cggcgccaac     120

ctcgagctgg tgttcccgtt catcgatgca cttgacccgg acctggaagt catcgccttc     180

gatgtgcctg gggtcggcgg ctcgtccaca ccacgccacc cgtatcgctt ccctgggctg     240

gccaagctga ccgcacgcat gctcgactac ctcgactacg gccaggtcaa cgtcatcggc     300

gtgtcctggg gcggcgccct ggcccagcag tttgctcacg attaccccga gcgctgcaag     360

aaactggtgc tggccgccac cgctgccggt gcggtaatgg tgccaggcaa gcccaaggtg     420

ctgtggatga tggccagccc ccggcgttac gtgcagccgt cgcatgtcat ccgcattgca     480

ccgatgatct atggcggcgg cttccgacgt gaccccgacc tggccatgca ccatgctgcc     540

aaagtgcgct ccggcggcaa gctgggctac tactggcagc tgttcgccgg gctgggctgg     600

accagcatcc actggctgca caagatccag cagcccaccc tggtactggc cggtgacgac     660

gacccgctga tcccgctgat caacatgcgc ctgctggcct ggcggattcc caatgcccag     720

ctacacatta tcgacgacgg ccatctgttc ctgatcaccc gtgccgaagc cgtcgccccg     780

atcatcatga agttcctgca ggaagaacgt cagcgtgcgg tcatgcatcc ccgtccggcc     840

tcgggggggt ga                                                         852